# EUROPÄISCHE PATENTANMELDUNG

(11) **EP 2 404 889 A1**
(43) Veröffentlichungstag der Anmeldung: **11.01.2012**
(21) Anmeldenummer: 10168550.1
(22) Anmeldetag: 06.07.2010
(51) Int. Cl.: C07C 29/149, C07C 29/80, C07C 29/94, C07C 31/20

(54) **Verfahren zur Herstellung von Alpha, Omega-Diolen**

(71) Anmelder: LANXESS Deutschland GmbH, 51369 Leverkusen (DE)
(72) Erfinder: Heuer, Lutz, 41542 Dormagen (DE)

(57) **Zusammenfassung**

Die vorliegende Erfindung betrifft ein Verfahren zur Herstellung und Reinigung von α,ω-Diolen wie insbesondere 1,6-Hexandiol (Adipol) unter Verwendung saurer Stoffe.

## Beschreibung

Die vorliegende Erfindung betrifft ein Verfahren zur Herstellung und Reinigung von α,ω-Diolen wie insbesondere 1,6-Hexandiol (Adipol) unter Verwendung saurer Stoffe.

α,ω-Diole wie beispielsweise 1,6-Hexandiol, im Folgenden auch Adipol genannt, wird in hohen Tonnagen für industrielle Anwendungen wie zum Beispiel der Herstellung von thermoplastischen Polyestern oder Polyurethanen eingesetzt. Die synthetische Herstellung von α,ω-Diolen ist prinzipiell bekannt.

So wird in WO 97/31882, US 6,407,294, US 6,706,932, US 5,981,769, DE 195 00 783, DE 196 07 954 A und GB 1 300 889 A und DE 1 023 750 A die Herstellung von α,ω-Diolen wie 1,4 Butandiol, 1,5-Pentandiol, Adipol ausgehend von den entsprechenden Dicarbonsäuren oder deren Estern bzw. ω-Hydroxycarbonsäuren, den entsprechenden Lactonen oder Estern von ω-Hydroxycarbonsäuren durch Hydrierung und anschließende destillative Aufreinigung beschrieben.

Allen vorgenannten Verfahren ist gemeinsam, dass in nicht unerheblichen Mengen anfallende Nebenprodukte wie insbesondere ω-Hydroxycarbonsäuren oder deren Ester gebildet werden, bei thermischer Belastung, wie sie bei der Aufreinigung der Zielprodukte durch Destillation typischerweise auftritt, zur Cyclisierung (Lactonbildung) neigen und damit während der Destillation fortlaufend Komponenten erzeugt werden, die aufgrund ihrer höheren Flüchtigkeit die Qualität der α,ω-Diole als Zielprodukte erheblich beeinträchtigen können.

In US 6,706,932 wird daher vorgeschlagen, die Bildung von Lactonen dadurch zu vermeiden, dass der Reaktionsmischung vor der Destillation alkalische Salze wie Hydroxide, Carbonate oder Hydrogencarbonate von Alkali- oder Erdalkalimetallen zugesetzt werden. Nachteilig an diesem Vorgehen ist der Umstand, dass sich im Kolonnensumpf die nichtflüchtigen Salze anreichern was die Verwertung der übrigen organischen Sumpfprodukte erschwert oder unmöglich macht.

In US 5,981,769 wird vorgeschlagen, die Bildung von Caprolacton in Strömen, die reich an 6-Hydroxycapronsäureestern sind, durch den Zusatz von Säuren, wie Schwefelsäure, Phosphorsäure oder Carbonsäuren zu fördern, um Caprolacton im Anschluss in einfacher Weise abdestillieren zu können.

Der Fachmann würde insofern generell davon absehen, Säuren zur Destillation zuzusetzen, um eine Verunreinigung der α,ω-Diole mit Lactonen zu vermeiden.

Es bestand daher weiterhin das Bedürfnis, ein Verfahren bereitzustellen, mit dem die aus der Hydrierung von Dicarbonsäuren oder deren Estern entstehenden α,ω-Diole in hoher Reinheit und weitestgehend ohne Verunreinigung mit analogen Lactonen erhalten werden können.

Es wurde nun ein Verfahren zur Herstellung von α,ω-Diolen der Formel (I) gefunden,

HO-(CH₂)ₙ-OH (I)

in der n für 4 bis 10, vorzugsweise 4 bis 8 und besonders bevorzugt für 4, 5 oder 6 und ganz bevorzugt für 6 steht
umfassend zumindest folgende Schritte
A) Hydrierung von
   ● Verbindungen der Formel (II)

      R¹OOC-(CH₂)₍ₙ₋₂₎-COOR² (II)

      in der die Reste R¹ und R² jeweils unabhängig voneinander, vorzugsweise jeweils identisch für Wasserstoff oder C₁-C₁₂-Alkyl wie beispielsweise Methyl, Ethyl, n-Propyl, n-Butyl, iso-Butyl, n-Hexyl, Cyclohexyl stehen,
   ● oder Verbindungen der Formeln (IIIa) oder (IIIb)

      H-[O-CR³-(CH₂)₍ₙ₋₂₎-CR⁴-]ₘ-OH (IIIa)

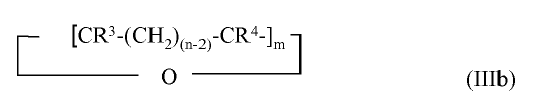

      in denen jeweils
      - R³ und R⁴: unabhängig voneinander für zwei Wasserstoffatome oder Carbonylsauerstoff mit der Maßgabe stehen, dass im gesamten Molekül zumindest eine Esterfunktionalität vorhanden ist und
      - m: für Verbindungen der Formel (IIIa) für eine ganze Zahl von 2 bis 45, bevorzugt 3 bis 40 steht und für Verbindungen der Formel (IIIb) für eine ganze Zahl von 2 bis 45, bevorzugt 3 bis 40 steht
      - n: die oben aufgeführte Bedeutung einschließlich ihrer Vorzugsbereiche besitzt
   ● oder beliebige Mischungen von Verbindungen der Formeln (II), (IIIa) und (IIIb),
   in Gegenwart eines Katalysators
B) destillative Reinigung der gemäß Schritt A) erhaltenen Reaktionsmischungen in einer ein-oder mehrstufigen Destillation, wobei gereinigte Verbindungen der Formel (I) als Kopf- oder Seitenstrom erhalten werden,
dadurch gekennzeichnet, dass die destillative Aufreinigung gemäß Schritt B) in Gegenwart von sauren Stoffen durchgeführt wird.

Der Rahmen der Erfindung umfasst neben den genannten Bereichen und Vorzugsbereichen von Formeln und Parametern auch beliebige Kombinationen davon, selbst wenn sie aus praktischen Gründen nachstehend nicht vollständig explizit aufgeführt sind.

Bevorzugte Verbindungen der Formel (I) sind Dimethyl- und Diethyladipat sowie entsprechende Ester technischer Alkoholgemische wie sie kommerziell beispielsweise von den Firmen BBASF SE und Ube erhältlich sind. Bevorzugte Verbindungen der Formeln (IIIa) und (IIIb) sind Adipoladipinate.

Unter dem Begriff Adipoladipinate sind sowohl monomere als auch oligomere und polymere Ester von 1,6-Hexandiol und 1,6-Hexandicarbonsäure zu verstehen.

Bevorzugte Adipoladipinate sind solche mit einem Molgewicht von 246 bis 10.000 oder Mischungen solcher Ester, besonders bevorzugt sind Adipoladipinate mit einem Molgewicht von 246 bis 4.000 und insbesondere Mischungen davon, wie zum Beispiel solche, die unter CAS 025212-06-0 geführt werden.

Gemäß Schritt A) werden die Verbindungen der Formeln (II), (IIIa) oder (IIIb) oder Mischungen davon in Gegenwart eines Katalysators hydriert.

Die katalytische Hydrierung erfolgt vorzugsweise durch Umsetzung mit molekularem Wasserstoff. Der Wasserstoffpartialdruck beträgt dabei beispielsweise 0,05 bis 50 MPa vorzugsweise 0,5 bis 40 MPa, besonders bevorzugt 5 bis 40 MPa und ganz besonders bevorzugt 25 bis 35 MPa. Vorzugsweise entspricht dies gleichzeitig zumindest ungefähr dem Reaktionsdruck.

Die Reaktionstemperatur beträgt beispielsweise 20 bis 400 °C, bevorzugt 100 bis 350°C und ganz besonders bevorzugt 190 bis 285°C.

Die Hydrierung kann homogen oder inhomogen erfolgen, wobei die inhomogene Hydrierung bevorzugt ist.

Die Hydrierung kann weiterhin kontinuierlich oder batchweise erfolgen, wobei eine kontinuierliche Verfahrensweise bevorzugt ist.

Besonders bevorzugt ist eine Verfahrensführung, in der die Hydrierung kontinuierlich an einem Festbettreaktor erfolgt.

Als Katalysatoren sind prinzipiell alle geeignet, die auch Estergruppen hydrieren können. Solche Katalysatoren sind dem Fachmann hinlänglich bekannt.

Geeignete Katalysatoren sind beispielsweise Übergangsmetallkatalysatoren. Bevorzugte Übergangsmetallkatalysatoren sind Übergangsmetallkatalysatoren enthaltend ein oder mehrere Übergangsmetalloxide oder deren reduzierte Formen, gegebenenfalls aufgebracht auf einem geeigneten Trägermaterial wie zum Beispiel Zirkondioxid, Titandioxid, Silikate wie Magnesium-oder Aluminiumsilikate, Zeolithen, Aluminiumoxiden, Siliciumdioxid, Graphit, Aktivkohlen oder Erdalkalimetallverbindungen sowie Mischungen dieser Trägermaterialien.

Solche bevorzugte Übergangsmetallkatalysatoren können stückig, als Presskörper, Pulver oder in einer anderen, dem Fachmann bekannten Haptik eingesetzt werden.

Besonders bevorzugte Übergangsmetallkatalysatoren sind solche, die in nichtreduziertem Zustand ein oder mehrere Metalloxide ausgewählt aus den Metallen Chrom, Molybdän, Wolfram, Kobalt, Rhenium, Kupfer, Mangan und Zink enthalten und vorzugsweise auf einem Trägermaterial aufgebracht sind.

Ganz besonders bevorzugte Übergangsmetallkatalysatoren sind beispielsweise solche, die im nicht reduzierten Zustand 20 bis 80 Gew.-% CuO, 10 bis 80 Gew.-% ZnO und als Trägermaterial 1 bis 50 Gew.-% Al₂O₃ enthalten oder solche, die im nicht reduzierten Zustand 60 bis 80 Gew.-% CuO, 2 bis 20 Gew.-% MnO und als Trägermaterial 1 bis 50 Gew.-% Al₂O₃ enthalten.

Noch weiter bevorzugte Katalysatoren sind solche die im nichtreduziertem Zustand 20 bis 80 Gew.-% CuO, 10 bis 80 Gew.-% ZnO und 1 bis 50 Gew.-% Al₂O₃ enthalten und durch Auffällung von Verbindungen des Kupfers und Zinks auf Aluminiumoxidpulver hergestellt wurden. In einer weiter bevorzugten Ausführungsform haben diese Katalysatoren eine Porengrößenverteilung bei der 5 bis 15% des Gesamtporenvolumens im Porendurchmesserbereich kleiner 150 Å und 80 bis 95 % im Porendurchmesserbereich größer 250 Å liegen, wobei die Porengrößenverteilung durch Quecksilberintrusion (Quecksilberporosimetrie) analog zu DIN 66133 unter Annahme eines zylindrischen Porenmodells bestimmt wird.

Die beschriebenen Katalysatoren können generell beispielsweise gemäß oder analog zu EP 552 463 A oder EP 1 338 587 A hergestellt werden.

Die als Edukte verwendeten Verbindungen der Formeln (II), (IIIa) oder (IIIb) können entweder in Substanz oder verdünnt mit einem Lösungsmittel hydriert werden. In einer bevorzugten werden die Verbindungen der Formeln (II), (IIIa) oder (IIIb) in Substanz kontinuierlich hydriert.

Weiterhin ist es möglich technische Estergemische zu hydrieren, die überwiegend die Verbindungen der Formeln(II), (IIIa) oder (IIIb) erhalten, jedoch weiterhin auch Beimengungen von Monoalkohole enthalten.

Gemäß Schritt A) wird eine Reaktionmischung erhalten, die
● im Wesentlichen Verbindungen der Formel (I) sowie
● Leichtsieder wie insbesondere Methylcyclopentanol-2, Lactone, und weitere leichtsiedende teilhydrierte Produkte sowie
● Schwersieder, wie insbesondere oligomere oder polymere teilhydrierte Produkte enthält.

Vorzugsweise enthält die gemäß schritt A) erhaltene Reaktionsmischung 60 bis 99 Gew.-% an Verbindungen der Formel (I), vorzugsweise 80 bis 98 Gew.-%, besonders bevorzugt 90 bis 96 Gew.-%.

Im Rahmen der Erfindung sind unter Leichtsiedern solche Verbindungen zu verstehen, die einen Siedepunkt besitzen, der bei gleichem Druck niedriger ist, als der der Verbindung der Formel (I), die über das erfindungsgemäße Verfahren gewonnen werden soll.

Unter Schwer- und Nichtsiedern sind demzufolge im Rahmen der Erfindung Verbindungen zu verstehen, die einen Siedepunkt besitzen, der bei gleichem Druck höher ist, als der der Verbindung der Formel (I), die über das erfindungsgemäße Verfahren gewonnen werden soll, bzw. solche Verbindungen, die sich nicht oder nicht chemisch unverändert destillieren lassen.

Die gemäß Schritt A) erhaltene Reaktionmischung wird gemäß Schritt B) in an sich grundsätzlich bekannter Weise destillativ gereinigt.

In einer Ausführungsform erfolgt die destillative Reinigung gemäß Schritt B) in zumindest zwei, bevorzugt zwei oder drei nacheinander geschalteten Destillationseinheiten durch Abtrennung der Leichtsieder in einer ersten Destillationseinheit, Abtrennung der Verbindungen der Formel (I) als Kopfstrom einer zweiten Destillationseinheit und gegebenenfalls Abtrennung von weiteren Anteilen an Verbindungen der Formel (I) als Kopfstrom in einer dritten Destillationseinheit.

Die erfindungsgemäße destillative Aufreinigung kann für jeden Schritt unabhängig batchweise oder kontinuierlich erfolgen, wobei die kontinuierliche destillative Aufreinigung bevorzugt ist.

Beispielsweise kann zur Reinigung eines Reaktionsgemisches, dass aus der Hydrierung von Adipoladipinat stammt, zunächst in einer ersten Kolonne bei einem Druck von 50 bis 100 mbar, einer Sumpftemperatur von 160 bis 200°C und einer Kopftemperatur von 60 bis 80°C zunächst Leichtsieder wie Methylcyclopentanol und 1-Hexanol entfernt werden, in einer zweiten Kolonne bei einem Druck von 30 bis 50 mbar, einer Sumpftemperatur von 150 bis 180°C und einer Kopftemperatur von 150 bis 170°C das gewünschte Adipol als Wertprodukt und in einer dritten Kolonne bei einem Druck von 1 bis 10 mbar, einer Sumpftemperatur von 160 bis 200°C und einer Kopftemperatur von 110 bis 140°C weiteres Wertprodukt neben Schwersiedern als Sumpfprodukt.

Zur destillativen Reinigung können alle dem Fachmann bekannten Destillationseinheiten wie insbesondere Kolonnen und Verdampfer verwendet werden.

Als Kolonnen können beispielsweise konventionelle einfache Kolonnen, Bodenkolonnen, Füllkörperkolonnen, Trennwandkolonnen, Seitenstromkolonnen oder thermisch gekoppelte Kolonnen eingesetzt werden, wobei die vorgenannten Kolonnen beliebige dem Fachmann bekannte Einbauten enthalten können.

In Bodenkolonnen können beispielsweise Sieb-, Glocken- oder Ventilböden eingebaut sein, auf denen die Flüssigkeit steht. Durch spezielle Schlitze oder Löcher kann Dampf in die Flüssigkeit eingeperlt werden, so dass eine Sprudelschicht entsteht.

Füllkörperkolonnen können mit unterschiedlichen Füllkörpern gefüllt werden, die eine gute Verteilung der Flüssigkeit und Verwirbelung der Gasströmung bewirken. Durch die Oberflächenvergrößerung der typischerweise etwa 5 bis 15 mm großen Partikel werden Wärme- und Stoffaustausch optimiert und die Trennfähigkeit der Kolonne somit erhöht. Bekannte Beispiele sind der Raschig-Ring (ein Hohlzylinder), Pall-Ring, Hiflow-Ring, Intalox-Sattel, Berl-Sattel und Igel. Die Füllkörper können geordnet, aber auch regellos (als Schüttung) in die Kolonne eingebracht werden. Als Materialien kommen in Frage Glas, Keramik, Metall und Kunststoffe.

Strukturierte Packungen sind eine Weiterentwicklung der geordneten Füllkörper. Sie weisen eine regelmäßig geformte Struktur auf. Dadurch ist es bei Packungen möglich, Einengungen für die Gasströmung (mit erheblichem Einfluss auf den Druckverlust) zu reduzieren. Es gibt verschiedene Ausführungen von Packungen z. B. Gewebe- oder Blechpackungen. Als Material können Metall, Kunststoff, Glas und Keramik je nach erforderlicher Benetzbarkeit eingesetzt werden.

Bevorzugt werden erfindungsgemäß Füllkörperkolonnen eingesetzt, in denen geschüttete Raschigringe verwendet werden.

Als Verdampfer können beispielsweise Dünnschicht- oder Fallfilmverdampfer eingesetzt werden.

In einer weiteren Ausführungsform kann die destillative Reinigung gemäß Schritt B) auch derart erfolgen, dass die Leichtsieder als Kopfstrom und die Verbindungen der Formel (I) als Seitenstrom in einer Seitenstromkolonne entnommen werden. Gegebenenfalls kann die Abtrennung von weiteren Anteilen an Verbindungen der Formel (I) als Kopfprodukt einer zweiten Kolonne entnommen werden.

Die destillative Reinigung gemäß Schritt B) wird erfindungsgemäß in Gegenwart von sauren Stoffen durchgeführt.

Unter sauren Stoffen sind im Rahmen der Erfindung solche Verbindungen zu verstehen, die einen pKs-Wert in Wasser bei 20°C oder berechnet auf ein wässriges Medium bei 20°C von weniger als 7,00, vorzugsweise als 6,50 aufweisen.

Beispiele für saure Stoffe umfassen anorganische und organische Säuren oder saure Salze mit einem pKs-Wert in Wasser bei 20°C von weniger als 7,00, vorzugsweise von 6,50 oder weniger sowie saure Ionentauscher, saure Tonerden oder Lewissäuren wie z.B. saure Oxide von Aluminium, Wolfram oder Vanadium.

Beispiele für saure Stoffe umfassen weiterhin auch Gase, die in Wasser Säuren mit einem pKs-Wert bei 20°C von weniger als 7,00, vorzugsweise weniger als 6,50 bilden, wie beispielsweise Kohlendioxid, Schwefeldioxid, Chlorwasserstoff und Schwefeltrioxid.

Bevorzugt sind anorganische und organische Säuren mit einem pKs-Wert in Wasser bei 20°C von weniger als 7,00, vorzugsweise von 6,50 oder weniger.

Beispiele für solche anorganischen Säuren sind Schwefelsäure, Phosphorsäure, Salzsäure, Beispiele für organische Säuren sind Monocarbonsäuren, Dicarbonsäuren und Tricarbonsäuren sowie Sulfonsäuren.

Besonders bevorzugt sind Monocarbonsäuren, Dicarbonsäuren und Tricarbonsäuren.

Ganz besonders bevorzugt werden eingesetzt
● Verbindungen der Formel (IIS)

   R¹OOC-(CH₂)₍ₙ₋₂₎-COOH (IIS)

   in der der Rest R¹ für Wasserstoff oder C₁-C₁₂-Alkyl wie beispielsweise Methyl, Ethyl, n-Propyl, n-Butyl oder iso-Butyl steht
● oder Verbindungen der Formel (IIIaS)

   H-[O-CR³-(CH₂)₍ₙ₋₂₎-CR⁴-]ₘ-OH (IIIaS)

   in der
   - R³ und R⁴: unabhängig voneinander für zwei Wasserstoffatome oder Carbonylsauerstoff mit der Maßgabe stehen, dass im gesamten Molekül zumindest eine Carbonsäurefunktion vorhanden ist und
   - m: für Verbindungen der Formel (IIIa) für eine ganze Zahl von 2 bis 45, bevorzugt 3 bis 40 steht und für Verbindungen der Formel (IIIb) für eine ganze Zahl von 2 bis 45, bevorzugt 3 bis 40 steht
   - wobei n: den gleichen Wert annimmt, wie für Verbindungen der Formel (I), die Zielprodukte des erfindungsgemäßen Verfahrens sind.

Bei der Herstellung von 1,4-Butandiol ist es dementsprechend ganz besonders bevorzugt Bernsteinsäure einzusetzen, bei der Herstellung von 1,5-Pentandiol Glutarsäure und bei der Herstellung von Adipol Adipinsäure.

Der Zusatz von sauren Stoffen kann nach der Durchführung des Schrittes A) in jedem Verfahrensschritt erfolgen, solange die Abtrennung der Verbindungen der Formel (I) von Schwersiedern in Gegenwart der sauren Stoffe stattfindet.

Vorzugsweise erfolgt der Zusatz von sauren Soffen dort, wo es verfahrenstechnisch aufgrund günstiger Rahmenparameter wie Druck, Temperatur oder Stromvolumen günstig ist, wie zum Beispiel im Rücklauf oder im Feedstrom der Kolonnen.

Grundsätzlich ist die Zugabe saurer Stoffe nur an einer Stelle z.B. im Kolonnenverbund erforderlich. Erfolgt sie am Anfang wird typischerweise eine Ansäuerung für alle folgenden Ströme der nachfolgenden Kolonnen beobachtet.

Die ist die Zugabe saurer Stoffe kann batchweise oder kontinuierlich erfolgen. Dabei können die sauren Stoffe entweder in Substanz, als Lösung, Dispersion, Emulsion oder Paste erfolgen, wobei vorzugsweise nur solche weiteren Stoffe Verwendung zur Bildung der Lösung, Dispersion, Emulsion oder Paste finden, die auch Bestandteil des gemäß Schritt A) erhaltenen Reaktionsgemisches sind.

Für die Herstellung von Adipol besteht eine bevorzugte Ausführungsform darin, Lösungen von 0,1 1 bis 25 Gew.-% Adipinsäure in Adipol einzusetzen vorzugsweise Lösungen von 5 bis 15 Gew.-% Adipinsäure in Adipol. Die Mischungen können weiterhin 0,1 bis 50 Gew.-% Wasser bezogen auf die Summe von Adipinsäure und Adipol enthalten.

Gleiches gilt analog für Glutarsäure und 1,5-Pentandiol sowie Bernsteinsäure und 1,4-Butandiol.

Es wurde gefunden, dass die vorgenannten Mischungen besonders vorteilhaft hinsichtlich Dosierbarkeit und der Vermeidung von unerwünschter Feststoffbildung in den Dosiereinrichtungen sind.

Die Menge an sauren Stoffen wird vorzugsweise so gewählt, dass bezogen auf die Menge des in Schritt B) destillativ zu reinigenden Reaktionsgemisches 0,001 bis 10 Gew.-%, vorzugsweise 0,002 bis 1 Gew.-% und besonders bevorzugt 0,005 bis 0,5 Gew.-% zugegeben werden.

Es sei angemerkt dass sich vorgenannte angaben naturgemäß nur auf die sauren Stoffe als solche, nicht jedoch auf miteingebrachte Lösungs- oder Hilfsmittel etc. bezieht.

In einer weiteren Ausführungsform erfolgt die Zugabe derart dass der jeweilige Kolonnensumpf einen pH-Wert bei 20°C von 4,5 bis 7,0, vorzugsweise 4,8 bis 6,5 aufweist, wobei die Messung des pH-Wertes derart erfolgt, dass eine Probe in Methanol/Wasser (Volumen 50:50) suspendiert wird (2 Gew.-%) und mit Hilfe einer kalibrierten pH-Einstabmeßkette der pH-Wert gemessen wird.

Erfindungsgemäß lässt sich die Bildung unerwünschter Lactone weitestgehend vermeiden, so dass sehr reine Verbindungen der Formel (I) erhalten werden können.

Typischerweise weisen die Verbindungen der Formel (I) erfindungsgemäß eine Reinheit von mehr als 98 Gew.-%, vorzugsweise 98,5 Gew.-% oder mehr und besonders bevorzugt 99,0 Gew.-% oder mehr auf.

Typischerweise liegt der Gehalt von Verbindungen der Formel (I) an analogen Lactonen der Formel (IV) 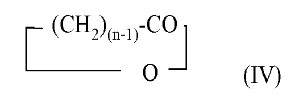 0,40 Gew.-% oder weniger, vorzugsweise 0,25 Gew.-% oder weniger.

Ein weiterer Vorteil der Erfindung ist darin zu sehen, dass trotz der Zugabe von Säuren die Bildung von Verbindungen der Formel (V)

HO-(CH2)₍ₙ₋₁₎-CHO (V)

weitestgehend unterdrückt wird.

Typischerweise liegt der Gehalt von Verbindungen der Formel (I) an Verbindungen der Formel (V) bei 0,05 Gew.-% oder weniger, vorzugsweise 0,005 Gew.-% oder weniger.

Die Erfindung sei anhand nachfolgender Beispiele ausgeführt, ohne ihren Gegenstand jedoch darauf zu beschränken.

### Beispiele

### Beispiel 1

72 g Aluminiumoxidpulver (spezifische Oberfläche 146,5 m²/g) wurde in einem Fällkessel in 41 Wasser suspendiert und auf 70°C temperiert. Aus einem Vorlagebehälter wurden 15 kg einer wässrigen Lösung, enthaltend 2628 g Cu(NO₃)₂ x 2,5 H₂O und 1200 g Zn(NO₃)₂ x 6 H₂O innerhalb von 3 Stunden in den Fällkessel gepumpt. Gleichzeitig wurden eine wässrige Sodalösung mit der Konzentration von 1 mol/l zugepumpt. Man stellte die Zugabegeschwindigkeit der Sodalösung so ein, dass der pH-Wert innerhalb des Bereichs von 6,8 bis 7 gehalten wurde. Die Fällung wurde bei einer Temperatur von 70°C durchgeführt. Im Anschluss an die Fällung ließ man die Suspension bei 70°C über einen Zeitraum von 2 Stunden nachrühren. Danach wurde filtriert und mit Wasser gewaschen. Der Filterkuchen wurde 12 Stunden bei 120°C im Vakuum getrocknet. Das getrocknete Material wurde anschließend über einen Zeitraum von 4 Stunden bei 480°C kalziniert. Das Kalzinierprodukt wurde gemahlen, mit 5 Gew.-% Graphit vermischt und auf einer Tablettenpresse zu Zylindern mit einer Höhe von 3 mm und einem Durchmesser von 5 mm tablettiert. Die Seitenbruchhärte betrug 121 N. Die spezifische Oberfläche (BET) betrug 24,0 m²/g. Die Seitenbruchhärte im reduzierten Zustand betrug 47 N. Das Gesamtporenvolumen betrug 191,9 mm³/g.

### Beispiel 2

Ein Reaktor aus Stahl, befüllt mit 4,5 t Katalysator, der analog zu Beispiel 1 hergestellt wurde, wurde bei einem Wasserstoffdruck von 280 bar und einer Temperatur von 265°C kontinuierlich mit 1370 kg Adipoladipinat (oligomerer Ester aus 1,6-Hexandiol und Adipinsäure mit Molekulargewichten von 246 bis 4000 g/mol und überwiegend endständigem Alkohol) beschickt. Es wurde eine Reaktionsmischung erhalten, die zu 94,2 % aus Adipol bestand und folgende weitere Komponenten aufwies: cis und trans-Methylcyclopentanol-2, 6-Hydroxyhexanal, Wasser, Hexanol, Pentanol, Butanol sowie schwersiedende teilhydriertn Adipoladipinaten.

### Beispiel 3 (zum Vergleich)

Die gemäß Beispiel 2 erhaltene Reaktionsmischung wurde ohne Zugabe von sauren Stoffen kontinuierlich über eine dreistufige destillative Aufarbeitung geführt, bei der im ersten Schritt Leichtsieder bei einem Druck von 80 mbar, einer Sumpftemperatur von 190°C und einer Kopftemperatur von 70°C entfernt wurden, in einer zweiten Kolonne bei einem Druck von 35 mbar, einer Sumpftemperatur von 180°C und einer Kopftemperatur von 166°C das gewünschte Adipol als Wertprodukt und in einer dritten Kolonne bei einem Druck von 6 mbar, einer Sumpftemperatur von 165°C und einer Kopftemperatur von 133°C weiteres Wertprodukt neben Schwersiedern als Sumpfprodukt.

Das so erhaltene Wertprodukt Adipol aus der dritten Kolonne wies einen Gehalt an Caprolacton von 0,92 Gew.-% auf, der pH-Wert des Kolonnensumpfes wurde mit 8,4 ermittelt.

### Beispiel 4

Völlig analog zu Beispiel 3 wurde die erhaltene Reaktionsmischung destillativ aufgearbeitet, wobei allerdings der Feedstrom der zweiten Kolonne mit einer Mischung von 78,6 Gew.-% Adipol, 8,6 Gew.-% Adipinsäure und 12,8 Gew.-% Wasser in einer Menge von 0,1 Gew.-% des Feedstroms versetzt wurde (d.h. die Menge des sauren Stoffs betrug 0,0086 Gew.-% bezogen auf die Menge des Feedstroms).

Das so erhaltene Wertprodukt Adipol vereinigt aus der dritten Kolonne wies einen Gehalt an Caprolacton von 0,19 Gew.-% auf, der pH-Wert des Kolonnensumpfes wurde mit 5,5 ermittelt.

## Patentansprüche

1. Verfahren zur Herstellung von α,ω-Diolen der Formel (I),
HO-(CH₂)ₙ-OH (I)
in der n für 4 bis 10 steht
umfassend zumindest folgende Schritte
A) Hydrierung von
● Verbindungen der Formel (II)
R¹OOC-(CH₂)₍ₙ₋₂₎-COOR² (II)
in der die Reste R¹ und R² jeweils unabhängig voneinander für Wasserstoff oder C₁-C₁₂-Alkyl stehen,
● oder Verbindungen der Formeln (IIIa) oder (IIIb)
H-[O-CR³-(CH₂)₍ₙ₋₂₎-CR⁴-]ₘ-OH (IIIa) 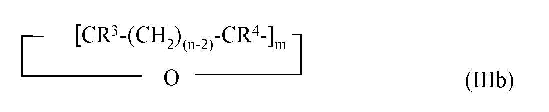
in denen jeweils
R³ und R⁴ unabhängig voneinander für zwei Wasserstoffatome oder Carbonylsauerstoff mit der Maßgabe stehen, dass im gesamten Molekül zumindest eine Esterfunktionalität vorhanden ist und
m für eine ganze Zahl von 2 bis 45 steht
wobei in den Formeln (II), (IIIa) und (IIIb)
n die oben aufgeführte Bedeutung besitzt
● oder beliebige Mischungen von Verbindungen der Formeln (II), (IIIa) und (IIIb),
in Gegenwart eines Katalysators
B) destillative Reinigung der gemäß Schritt A) erhaltenen Reaktionsmischungen in einer ein- oder mehrstufigen Destillation, wobei gereinigte Verbindungen der Formel (I) als Kopf- oder Seitenstrom erhalten werden,
**dadurch gekennzeichnet, dass** die destillative Aufreinigung gemäß Schritt B) in Gegenwart von sauren Stoffen durchgeführt wird.

2. Verfahren nach Anspruch 1, **dadurch gekennzeichnet, dass** als Verbindungen der Formel (II) Dimethyl- oder Diethyladipat oder als Verbindungen der Formeln (IIIa) und (IIIb) Adipoladipinate eingesetzt werden.

3. Verfahren nach Anspruch 1 oder 2, **dadurch gekennzeichnet, dass** die katalytische Hydrierung durch Umsetzung mit molekularem Wasserstoff bei einem Wasserstoffpartialdruck von 0,05 bis 50 MPa erfolgt.

4. Verfahren nach einem der Ansprüche 1 bis 3, **dadurch gekennzeichnet, dass** die Hydrierung kontinuierlich erfolgt.

5. Verfahren nach einem der Ansprüche 1 bis 4, **dadurch gekennzeichnet, dass** die Hydrierung an einem Festbettreaktor erfolgt.

6. Verfahren nach einem der Ansprüche 1 bis 5, **dadurch gekennzeichnet, dass** die destillative Reinigung gemäß Schritt B) in zumindest zwei nacheinander geschalteten Destillationseinheiten erfolgt.

7. Verfahren nach einem der Ansprüche 1 bis 6, **dadurch gekennzeichnet, dass** die destillative Reinigung kontinuierlich erfolgt.

8. Verfahren nach einem der Ansprüche 1 bis 7, **dadurch gekennzeichnet, dass** als saure Stoffe anorganische und organische Säuren oder saure Salze mit einem pKs-Wert in Wasser bei 20°C von weniger als 7,00 eingesetzt werden.

9. Verfahren nach einem der Ansprüche 1 bis 8, **dadurch gekennzeichnet, dass** als saure Stoffe folgende eingesetzt werden:
● Verbindungen der Formel (IIS)
R¹OOC-(CH₂)₍ₙ₋₂₎-COOH (IIS)
in der der Rest R¹ für Wasserstoff oder C₁-C₁₂-Alkyl steht
● oder Verbindungen der Formel (IIIaS)
H-[O-CR³-(CH₂)₍ₙ₋₂₎-CR⁴-]ₘ-OH (IIIaS)
in der
R³ und R⁴ unabhängig voneinander für zwei Wasserstoffatome oder Carbonylsauerstoff mit der Maßgabe stehen, dass im gesamten Molekül zumindest eine Carbonsäurefunktion vorhanden ist und
m für eine ganze Zahl von 2 bis 45 steht
wobei n den gleichen Wert annimmt, wie für Verbindungen der Formel (I), die Zielprodukte des Verfahrens nach den Ansprüchen 1 bis 8 sind.

10. Verfahren nach einem der Ansprüche 1 bis 9, **dadurch gekennzeichnet, dass** für den Fall dass n = 4, 5 oder 6 ist der saure Stoff in Form von Lösungen von 0,1 bis 25 Gew.-% Bernsteinsäure in 1,4-Butandiol (für n=4), Glutarsäure in 1,5-Pentandiol (für n=5) oder Adipinsäure in Adipol (für n=6) eingesetzt werden, wobei die Mischungen weiterhin 0,1 bis 50 Gew.-% Wasser bezogen auf die Summe von Bernsteinsäure und 1,4-Butandiol (für n=4) bzw. Glutarsäure und 1,5-Pentandiol (für n=5) oder Adipinsäure und Adipol (für n=6) enthalten können.

11. Verfahren nach einem der Ansprüche 1 bis 10, **dadurch gekennzeichnet, dass** die Menge an sauren Stoffen so gewählt wird, dass bezogen auf die Menge des in Schritt B) destillativ zu reinigenden Reaktionsgemisches 0,001 bis 10 Gew.-% zugegeben werden.

12. Verfahren nach einem der Ansprüche 1 bis 11, **dadurch gekennzeichnet, dass** die Zugabe von sauren Stoffe derart erfolgt, dass der bei der destillativen Reinigung auftretende Kolonnensumpf einen pH-Wert bei 20°C von 4,5 bis 7,0 aufweist.

13. Verfahren nach einem der Ansprüche 1 bis 14, **dadurch gekennzeichnet, dass** der Gehalt von Verbindungen der Formel (I) an analogen Lactonen der Formel (IV) 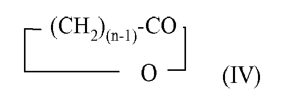 0,40 Gew.-% oder weniger beträgt..

14. Verwendung von Verbindungen der Formeln (IIS) und (IIIaS) gemäß Anspruch 9 zur destillativen Reinigung von Verbindungen der Formel (I) gemäß Anspruch 1.
